# EUROPEAN PATENT APPLICATION

(11) **EP 1 918 270 A1**
(43) Date of publication of application: **07.05.2008**
(21) Application number: 06757218.0
(22) Date of filing: 09.06.2006
(51) Int. Cl.: C07C 46/10, B01J 20/26, C07C 50/06, C12P 7/66

(54) **METHOD OF PURIFYING UBIQUINONE-10**

(30) Priority: 10.06.2005 JP 2005170939
(71) Applicant: KYOWA HAKKO KOGYO CO., LTD., Tokyo 100-8185 (JP); NIPPON RENSUI CO., Toshima-ku Tokyo 170-0005 (JP)
(72) Inventor: NAGANO, Hiroshi c/o Technical Research Laboratories, Hofu-shi Yamaguchi 747-8522 (JP); YAMANASHI, Hiroto c/o Technical Research Laboratories, Hofu-shi Yamaguchi 747-8522 (JP); MURATA, Hideki c/o Hofu Plant, Hofu-shi Yamaguchi 747-8522 (JP)
(74) Representative: Duckworth, Timothy John
(86) International application number: PCT/JP2006/311595
(87) International publication number: WO 2006/132348

(57) **Abstract**

According to the present invention, a method of purifying ubiquinone-10, which includes separating and removing a substance structurally similar to ubiquinone-10 contained in a starting material solution containing ubiquinone-10 by the use of a porous crosslinked acrylic polymer, more preferably, a method of purifying ubiquinone-10 by separating and removing a substance structurally similar to ubiquinone-10 contained in a starting material solution, which includes feeding a starting material solution containing ubiquinone-10 in a simulated moving bed chromatography apparatus wherein a solution can circulate in one direction in a packed-bed wherein a porous crosslinked acrylic polymer is packed, and flowing an eluent in the packed-bed in one direction, is provided.

## Description

### Technical Field

The present invention relates to a purification method of ubiquinone-10.

### Background Art

Ubiquinone-10 is widely present in the tissues of animals and plants as well as the cells of microorganisms, and plays an important role as an essential component of the terminal electron transport system. The pharmacological action of ubiquinone-10 is effective for congestive heart failure, coronary insufficiency, muscular dystrophy due to malnutrition, and the like. It is also a highly valuable substance as a pharmaceutical product.
As a production method of ubiquinone-10, a method comprising culturing a microorganism having a high ubiquinone-10 content and extracting ubiquinone-10 from the obtained culture, as well as synthetic methods are known.

As a method of purifying ubiquinone-10 from a solution containing ubiquinone-10 extracted from the culture, a method using silica gel or active alumina as known (see patent reference 1 and 2).
However, the extract obtained by the above-mentioned extraction method contains, besides ubiquinone-10, a large amount of a substance structurally similar to ubiquinone-10. Purification of ubiquinone-10 with a high purity directly from the extract by a crystallization method is difficult.

As a method of separation and purification of ubiquinone-10 with a high purity from a culture containing ubiquinone-10, a method including direct crystallization of ubiquinone-10 from a solution extracted with methanol is known (see patent reference 3).
However, ubiquinone-10 cannot be efficiently separated and purified by applying the extract containing a large amount of a substance structurally similar to ubiquinone-10 to a method using silica gel or active alumina. Moreover, silica gel and active alumina are expensive, which problematically results in high production cost on an industrial scale. Moreover, since the solubility of ubiquinone-10 in methanol is as low as 5 g/L or below, the above-mentioned methanol extraction method requiring a large amount of methanol is not an environmentally desirable method.

It is known that ubiquinone-10 can be separated and purified from a liposoluble substance-containing sample comprising ubiquinone-10, vitamin K1 and vitamin K2 using a copolymer of methyl methacrylate and ethyleneglycol dimethacrylate (non-patent reference 1). However, it is not known that ubiquinone-10 can be purified by separating ubiquinone-10 from a substance structurally similar to ubiquinone-10 using the polymer.

While a simulated moving bed separation method is known as a method for separating a solution containing two or more components into two fractions based on the difference in the moving rates of respective components (non-patent reference 2), it is not known that ubiquinone-10 and a substance structurally similar to ubiquinone-10 can be separated by the simulated moving bed separation method.
patent reference 1: JP-A-63-91360
patent reference 2: JP-A-1-160953
patent reference 3: W02004/011660
non-patent reference 1: DIAION material, NIPPON RENSUI CO., Mitsubishi Chemical Corporation
non-patent reference 2: high purification technique, vol. 2 separation technique, Fujitec Corporation

### Disclosure of the Invention

### Problems to be Solved by the Invention

An object of the present invention is to provide a purification method of ubiquinone-10.

### Means of Solving the Problems

The present invention relates to the following (1) - (6).
(1) A method of purifying ubiquinone-10, which comprises separating and removing a substance structurally similar to ubiquinone-10 contained in a starting material solution containing ubiquinone-10 by the use of a porous crosslinked acrylic polymer.
(2) A method of purifying ubiquinone-10 by separating and removing a substance structurally similar to ubiquinone-10 contained in a starting material solution, which comprises feeding a starting material solution containing ubiquinone-10 in a simulated moving bed chromatography apparatus in which a solution can circulate in one direction in a packed-bed wherein a porous crosslinked acrylic polymer is packed, and moving an eluent in the packed-bed in one direction.
(3) The method of the above-mentioned (1) or (2), wherein the porous crosslinked acrylic polymer is a spherical polymer particle having an average particle size of 10 - 500 µm.
(4) The method of any one of the above-mentioned (1) to (3), wherein the porous crosslinked acrylic polymer is a polymer of methacrylic acid ester and ethyleneglycol dimethacrylate.
(5) The method of any one of the above-mentioned (1) to (4), wherein the starting material solution containing ubiquinone-10 is an extract of a culture of a microorganism having an ability to produce ubiquinone-10, or a crude purified product of ubiquinone-10.
(6) The method of any one of the above-mentioned (1) to (5), wherein the substance structurally similar to ubiquinone-10 is 5-demethoxyubiquinone-10.

### Effect of the Invention

According to the present invention, ubiquinone-10 can be purified efficiently.

### Brief Description of the Drawings

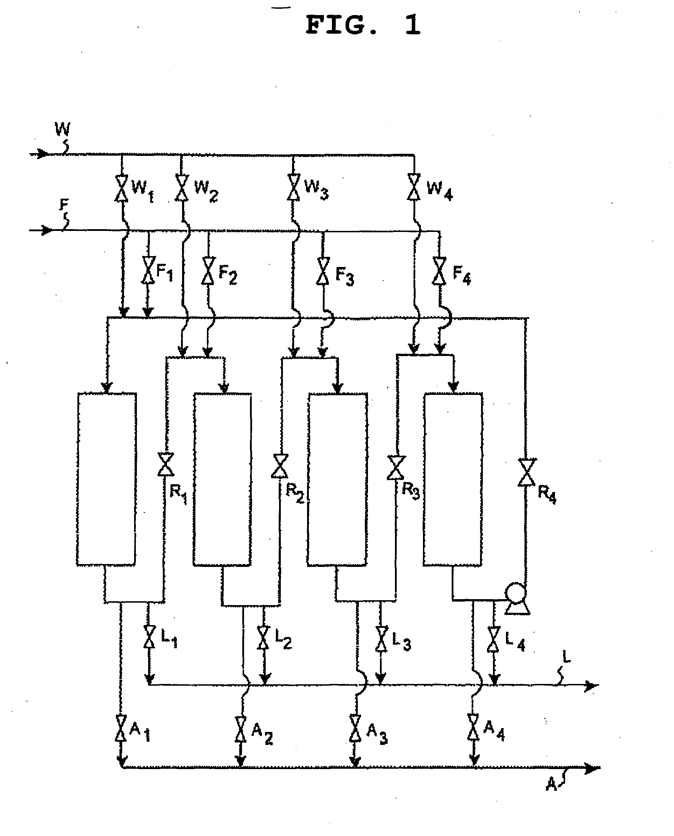

[Fig. 1] Fig. 1 shows a simulated moving bed separation apparatus.

### Explanation of symbols

W: eluent feed pipe
F: starting material solution feed pipe
L: 5-demethoxyubiquinone-10 solution withdrawal pipe
A: ubiquinone-10 solution withdrawal pipe
W₁ - W₄: valve
F₁ - F₄: valve
L₁ - L₄: valve
A₁ - A₄: valve
R₁ - R₄: check valve

### Best Mode for Embodying the Invention

### 1. Porous crosslinked acrylic polymer to be used for the present invention

The porous crosslinked acrylic polymer to be used for the method of the present invention is not particularly limited as long as it shows different affinity for ubiquinone-10 and a substance structurally similar to ubiquinone-10. Preferred is a polymer having an average particle size of 10 - 500 µm, preferably 10 - 200 µm, more preferably 10 - 100 µm.

In addition, examples of the porous crosslinked acrylic polymer to be used for the method of the present invention include a crosslinked polymer of methacrylic acid ester such as methyl methacrylate and ethyleneglycol dimethacrylate, and the like, such as DIAION HP2MG (manufactured by Mitsubishi Chemical Corporation), which is a crosslinked polymer of methyl methacrylate and ethyleneglycol dimethacrylate, and the like.

### 2. Starting material solution containing ubiquinone-10

The starting material solution containing ubiquinone-10 is not particularly limited as long as it is a solution containing ubiquinone-10 and a substance structurally similar to ubiquinone-10. Examples thereof include a starting material solution containing ubiquinone-10, which contains a substance structurally similar to ubiquinone-10 at a weight ratio of not more than 10%, preferably not more than 5%, more preferably not more than 3%, still more preferably not more than 2%, relative to ubiquinone-10, when analyzed by high performance liquid chromatography (HPLC). The analysis conditions of HPLC are, for example, those described in the following 4.

Examples of the starting material solution containing ubiquinone-10 include an extract of a culture of a microorganism having an ability to produce ubiquinone-10, a crude purification product of ubiquinone-10 and the like.
A culture of a microorganism having an ability to produce ubiquinone-10 can be obtained by culturing a microorganism having an ability to produce ubiquinone-10 by a conventional method.

The microorganism having an ability to produce ubiquinone-10 may be any microorganism as long as it has such ability. For example, basidiomycetes, fungi, yeasts and bacteria known to be microorganisms producing ubiquinone-10 can be employed. More specifically, basidiomycetes include microorganisms belonging to the genus Ustilago, fungi include microorganisms belonging to the genus Aspergillus, the genus Exobasidium, the genus Geotrichum, the genus Monascus, the genus Paecilomyces, the genus Sporotrichum and the genus Tilletiopsis, yeast include microorganisms belonging to the genus Aureobasidium, the genus Brettanomyces, the genus Bullera, the genus Candida, the genus Cryptococcus, the genus Leucosporidium, the genus Oosporidium, the genus Rhodotorula, the genus Rhodosporium, the genus Schizosaccharomyces, the genus Sporobolomyces, the genus Torulopsis, the genus Tremella, the genus Trichosporon and the genus Sporidiobolus, bacteria include microorganisms belonging to the genus Acetobacter, the genus Agrobacterium, the genus Corynebacterium, the genus Erythrobacter, the genus Flavobacterium, the genus Methylobacter, the genus Microcyclus, the genus Paracoccus, the genus Phyllobacterium, the genus Protaminobacter, the genus Pseudomonas, the genus Rhizobium, the genus Rhodobacter and the genus Xantomonas, and the like. Preferred are microorganisms belonging to the genus Rhodobacter, more preferred is Rhodobacter sphaeroides.

In addition, microorganisms belonging to the genus Escherichia with ubiquinone synthesizing enzyme strengthened by genetic engineering and the like, and the above-mentioned microorganisms having an ability to produce ubiquinone-10, wherein the enzyme is strengthened, can also be used for the method of the present invention.
A medium used for culturing the above-mentioned microorganisms may be a natural medium or a synthetic medium as long as it contains a carbon source, a nitrogen source, inorganic salts and the like to be utilized by the microorganisms, and enables efficient culturing of the microorganisms.

The carbon source may be any as long as the microorganisms can utilize, and glucose, fructose, sucrose, molasses containing them, carbohydrates such as starch and starch hydrolysate, organic acid such as acetic acid and propionic acid, alcohols such as ethanol and propanol, and the like can be used.
As the nitrogen source, ammonium salts of inorganic acid or organic acid such as ammonia, ammonium chloride, ammonium sulfate, ammonium acetate and ammonium phosphate, other nitrogen-containing compounds, peptone, meat extract, yeast extract, corn steep liquor, casein hydrolysate, soybean cake and soybean cake hydrolysate, various fermentation microorganisms and digests thereof, and the like can be used.

As the inorganic salt, potassium dihydrogenphosphate, potassium monophosphate, magnesium phosphate, magnesium sulfate, sodium chloride, ferrous sulfate, manganese sulfate, copper sulfate, calcium carbonate and the like can be used.
Culturing is performed under aerobic conditions by, for example, shaking culture, submerged culturing with agitation and aeration and the like. The culturing temperature is preferably 15°C - 40°C and the culturing time is generally 16 hr - 14 days. The pH is preferably maintained at 3.0 - 9.0 during culturing. The pH is adjusted with inorganic or organic acid, alkali solution, urea, calcium carbonate, ammonia and the like.

After the completion of culturing, a solvent is added to a culture, microbial cells obtained by centrifugation of the culture, or dried microbial cells obtained by spray drying the microbial cells etc., the mixture is stirred for a time sufficient for extraction of ubiquinone-10, preferably 1 - 3 hr, and a fraction containing ubiquinone-10 is separated as a solvent layer or a solution layer free of an insoluble material such as microbial cells, whereby an extract of a culture of a microorganism having an ability to produce ubiquinone-10 to be used in the present invention can be obtained.

The above-mentioned solvent may be a lipophilic solvent, a hydrophilic solvent or a mixture thereof as long as it can extract ubiquinone-10 from a culture of a microorganism having an ability to produce ubiquinone-10. Examples of the lipophilic solvent include hexane, heptane and the like, more preferably hexane, examples of the hydrophilic solvent include methanol, ethanol, acetone and the like, more preferably ethanol, and examples of the mixture of a lipophilic solvent and a hydrophilic solvent include any combination of the above-mentioned lipophilic solvent and hydrophilic solvent.

The mixing ratio of a lipophilic solvent and a hydrophilic solvent in a mixture thereof may be any as long as it permits extraction of ubiquinone-10 from a culture of a microorganism having an ability to produce ubiquinone-10. The mixing volume ratio of a lipophilic solvent and a hydrophilic solvent is preferably 1000:1 - 1:1, more preferably 500:1 - 1:1, still more preferably 200:1 - 2:1, particularly preferably 100:1 - 3:1, most preferably 50:1 - 4:1.

Examples of a crudely purified product of ubiquinone-10 include a dried product obtained by drying or freeze-drying an extract obtained by extracting ubiquinone-10 with a solvent and the like from a culture obtained by culturing a microorganism having an ability to produce ubiquinone-10, and a solution obtained by dissolving, in a solvent, a crystal obtained by crystallization of an extract obtained by the extraction method. The solvent used for dissolution may be any as long as it can dissolve ubiquinone-10. Preferred are solvents usable for extraction of ubiquinone-10 from a culture of the above-mentioned microorganism having an ability to produce ubiquinone-10, and more preferred is a solvent having the same composition as those of the separation liquid mentioned below.

Examples of the substance structurally similar to ubiquinone-10 contained in a starting material solution containing ubiquinone-10 include 5-demethoxyubiquinone-10, 6-methoxy-2-polyprenyl1,4-benzoquinone, 6-methoxy-2-polyprenylphenol and the like, with preference given to 5-demethoxyubiquinone-10.

### 3. Purification method of ubiquinone-10 of the present invention

### (1) Single column separation method

A starting material solution containing ubiquinone-10 is fed from the upper end of a packed-bed containing a porous crosslinked acrylic polymer packed as a separation agent, then an eluent is also fed from the upper end, thus alternately separating respective components of the starting material solution. As a result, a ubiquinone-10 fraction having weak affinity for the separation agent can be first obtained from the lower part of the packed-bed, and then a fraction of a substance structurally similar to ubiquinone-10 such as 5-demethoxyubiquinone-10 and the like, which has stronger affinity for the separation agent than ubiquinone-10 can be obtained.

As the conditions (composition of eluent, temperature and the like) necessary for practicing the single column separation method, the following conditions described in the simulated moving bed separation method can be mentioned.

### (2) Simulated moving bed separation method.

For a simulated moving bed separation method, any can be used such as a commercially available simulated moving bed separation apparatus and a simulated moving bed separation apparatus as long as the anterior end and the posterior end of a packed-bed are connected by a solution channel and the solution can circulate in the apparatus.

A specific example of the simulated moving bed apparatus is an apparatus comprising a packed-bed comprising, along the flow of solutions, 4 kinds of feed ports and withdrawal ports consisting of a feed port of a starting material solution, a withdrawal port to withdraw a concentrated solution of a component with weak affinity for a separation agent, an eluent feed port, and a withdrawal port to withdraw a concentrated solution of a component with strong affinity for the separation agent in this order, wherein the feed port and withdrawal port to be in operation are, along with the shift of concentration distribution of each component, which is formed in the packed-bed, periodically switched to the feed port and withdrawal port at the downstream while maintaining the relative positional relationship thereof.

The packed-bed of a simulated moving bed is packed with a porous crosslinked acrylic polymer as a separation agent, which is divided into 4 zones based on functions thereof: an adsorption zone between the feed port of a starting material solution and the withdrawal port of a concentrated solution of a component with weak affinity, a purification zone between the withdrawal port and the eluent feed port, a desorption zone between the eluent feed port and the withdrawal port of a concentrated solution of a component with strong affinity, a concentration zone between the withdrawal port and the feed port of a starting material solution. These 4 zones sequentially move in the downstream direction as a result of the change of the feed port and withdrawal port to be in operation.

The simulated moving bed method to be used in the present invention may be based on a method including separating respective components from each other by constantly circulating a solution in a packed-bed, feeding a starting material solution containing ubiquinone-10 (hereinafter to be also simply referred to as a starting material solution) and an eluent into the circulation flow, and simultaneously withdrawing a part thereof from the circulation flow at each withdrawal port, a method individually performing mutual separation of components by circulation of a solution, feeding of a starting material solution and an eluent, and withdrawing of a ubiquinone-10 solution and a substance structurally similar to ubiquinone-10 solution (JP-A-2-49159) and the like. Preferred is the latter method.

To be specific, the method includes performing a process consisting of a feed-withdrawal step comprising feeding a starting material solution containing ubiquinone-10 and an eluent from each feed port into a packed-bed, withdrawing, from the packed-bed, a part of the solution that reached the withdrawal port of the ubiquinone-10 solution, and withdrawing, from the packed-bed, the total amount of the solution that reached the withdrawal port of the substance structurally similar to ubiquinone-10 solution, and a circulation step comprising moving the solutions in the packed-bed in the downstream direction without feeding the starting material solution and the eluent into the packed-bed and withdrawing of the solution from the packed-bed; then changing the feed port and withdrawal port to be in operation to the feed port and withdrawal port at the downstream while maintaining the relative positional relationship; and performing the above-mentioned steps again.

By repeating in this way the process consisting of the feed-withdrawal step and the circulation step and change of the feed port and withdrawal port to be in operation, the starting material solution can be separated into a ubiquinone-10 solution and a substance structurally similar to ubiquinone-10 solution. According to this method, good separation performance can be achieved even using a simple apparatus such as a simulated moving bed consisting of 4 unit packed-beds.

According to any method, the starting material solution contains, for example, ubiquinone-10 at a concentration of 10 - 300 g/L, preferably 20 - 200 g/L, more preferably 30 - 100 g/L, still more preferably 40 - 80 g/L. While the temperature of the starting material solution and eluent is not particularly limited as long as ubiquinone-10 can be efficiently purified, preferred is a temperature at which a solution within the packed-bed shows a low viscosity and ubiquinone-10 is not precipitated as a crystal. For example, 20°C - 50°C, preferably 23°C - 28°C, more preferably about 25°C, can be employed.

The starting material solution is subjected to a precolumn treatment as necessary and preferably fed into an apparatus after removing a substance irreversively adsorbed to a porous crosslinked acrylic polymer. The porous crosslinked acrylic polymer to be used for a precolumn treatment may be any as long as it has weak adsorbability to ubiquinone-10. Preferred is a polymer same as the porous crosslinked acrylic polymer to be used for the single column separation method or simulated moving bed separation method to be used in the subsequent step.

An eluent is generally used in a 2- to 10-fold amount, preferably 3- to 5-fold amount, relative to the starting material solution.
The eluent may be any as long as it can separate ubiquinone-10 from a substance structurally similar to ubiquinone-10, and a lipophilic solvent, a hydrophilic solvent and a mixture thereof can be mentioned. Examples of the lipophilic solvent include hexane, heptane and the like, examples of the hydrophilic solvent include methanol, ethanol, acetone and the like, and examples of the mixture of a lipophilic solvent and a hydrophilic solvent include a mixture of any combination of the above-mentioned lipophilic solvent and hydrophilic solvent.

When the substance structurally similar to ubiquinone-10 is 5-demethoxyubiquinone-10, a lipophilic solvent is preferably hexane, a hydrophilic solvent is preferably ethanol, and a mixture of a lipophilic solvent and a hydrophilic solvent is preferably that of hexane and ethanol.
The mixing ratio of a lipophilic solvent and a hydrophilic solvent in a mixture thereof may be any as long as it can efficiently separate ubiquinone-10 and a substance structurally similar to ubiquinone-10. For example, the mixing volume ratio of a lipophilic solvent and a hydrophilic solvent is preferably 1000:1 - 1:1, more preferably 500:1 - 1:1, still more preferably 200:1 - 2:1, particularly preferably 100:1 - 2:1, most preferably 50:1 - 10:1.

When the mixture of a lipophilic solvent and a hydrophilic solvent is a mixture of hexane and ethanol, the mixing volume ratio of hexane and ethanol is particularly preferably 40:1 - 20:1.
The linear velocity of the eluent may be any as long as it can efficiently separate a ubiquinone-10 from a substance structurally similar to ubiquinone-10 using the above-mentioned separation solution. Preferably, it is 0.5 - 10.0 m/h, more preferably 0.7 - 5.0 m/h, still more preferably 1.0 - 2.0 m/h.

### 4. Ubiquinone-10 obtained by the method of the present invention

The ubiquinone-10 obtained by the method of the present invention may be one wherein the wt% of the substance structurally similar to ubiquinone-10 contained as impurity in a sample by the analysis of a ubiquinone-10 containing sample under the following HPLC conditions is not more than 0.05%, preferably not more than 0.03%, more preferably not more than 0.02%.
HPLC analysis conditions:
Column: ZORBAX SIL 4.6 mm I.D. ×250 mm (manufactured by GL Science)
Eluent composition: cyclohexane:diethyl ether =4:1
Flow rate: 0.5 ml/min
Temperature: 25°C
Detection: UV 275 nm

The present invention is specifically explained in the following Examples, which are not to be construed as limitative.

### Example 1

### Preparation of starting material solution containing ubiquinone-10

A medium having the composition described in the following Table 1 was adjusted to pH 9.0, calcium carbonate was added to 1%, and the mixture was sterilized at 121°C for 10 min. The medium (1.8 L) was placed in a 3 L fermenter, and a ubiquinone-10 producing strain (Rhodobacter sphaeroides ATCC21286) was inoculated thereto and cultured at 28°C, stirring rotation 450 rpm for 8 days. In the Table, the trace element means a solution containing 88 mg/l sodium tetraborate (borax, Na₂B₄O₇·10H₂O), 37 mg/l ammonium molybdate [(NH₄)₆Mo₇O₂₄· 4H₂O], 8.8 mg/l zinc sulfate (ZnSO₄), 270 mg/l copper sulfate (CuSO₄·5H₂O), 7.2 mg/l manganese chloride (MnCl₂·4H₂O) and 970 mg/l ferric chloride (FeCl₃·6H₂O).

**[Table 1]**

| composition | concentration |
|---|---|
| blackstrap molasses | 4.0 % |
| glucose | 2.7 % |
| corn steep liquor | 4.0 % |
| ammonium sulfate potassium | 0.8 % |
| dihydrogenphosphate | 0.05 % |
| potassium monophosphate magnesium sulfate·7 | 0.05 % |
| hydrate | 0.025% |
| ferrous sulfate·7 hydrate | 3.0 mg/L |
| thiamine | 8.0 mg/L |
| nicotinic acid | 8.0 mg/L |
| trace element | 1.0 mL/L |

After the completion of the culturing, the microbial cells were recovered by centrifugation and dried with a spray dryer to give dried microbial cells containing ubiquinone-10. A 10-fold amount of ethanol was added to the dried microbial cells, and the mixture was stirred at 60°C for 3 hr to give an ethanol solution containing ubiquinone-10. The obtained ethanol solution was concentrated and cooled to 15°C to allow crystal precipitation of ubiquinone-10, whereby a crudely purified product containing ubiquinone-10 was obtained.

The crudely purified product was dissolved in a hexane:ethanol=25:1 solution, and applied to a precolumn (manufactured by Mitsubishi Chemical Corporation) packed with a porous crosslinked polymer (DIAION HP2MG) of methyl methacrylate and ethyleneglycol dimethacrylate. Irreversibly adsorbed components were removed and a resulting solution was used as a starting material solution containing ubiquinone-10. The starting material solution was analyzed by HPLC under the following conditions. As a result, it was confirmed that the starting material solution contained 48 g/L ubiquinone-10 and 0.4 g/L 5-demethoxyubiquinone-10.
HPLC analysis conditions:
Column: ZORBAX SIL 4.6 mm I.D. ×250 mm (GL Science)
Eluent composition: cyclohexane:diethyl ether =4:1
Flow rate: 0.5 ml/min
Temperature: 25°C
Detection: UV 275 nm

### Example 2

### Purification of ubiquinone-10 using a simulated moving bed separation apparatus

As the simulated moving bed separation apparatus, a compact chromatoseparation apparatus Labo fine (manufactured by NIPPON RENSUI CO.) consisting of 4 unit packed-beds of inner diameter 20 mm, packing layer height 250 mm, wherein the configuration is equipped with a starting material solution feed pipe F, an eluent feed pipe W, a ubiquinone-10 solution withdrawal pipe A, and a 5-demethoxyubiquinone-10 solution (substance structurally similar to ubiquinone-10) withdrawal pipe L, as shown in Fig. 1, was used.

Each unit packed-bed was packed with a porous crosslinked polymer (DIAION HP2MG, average particle size 75 µm, manufactured by Mitsubishi Chemical Corporation) of methyl methacrylate and ethyleneglycol dimethacrylate.
The starting material solution containing ubiquinone-10, obtained in Example 1, and an eluent which was a mixture of hexane:ethanol=25:1 were fed to the above-mentioned simulated moving bed separation apparatus at 25°C, and an operation including a 11 min feed-withdrawal step to withdraw a ubiquinone-10 solution and a 5-demethoxyubiquinone-10 solution and a 15 min circulation step to circulate the internal solution without feed-withdrawal was repeated while sequentially changing the feed port and withdrawal port to the corresponding feed port and withdrawal port of the unit packed-bed located directly downstream thereof. Accordingly, when the process is repeated 4 times, it returns to the original state. The feed rate of the starting material solution and eluent in the feed-withdrawal step, the solution withdrawing rate, and the circulation solution flow rate (solution sending flow rate of circulation pump) in the circulation step were all 360 ml/h (linear velocity 1.15 m/h).

The opening and closing of the valves during the period of 4 repeats of the process up to return to the original state of the apparatus were as shown in the following Table 2, wherein ○ shows open valve and × shows closed valve. The concentrations of the starting material solution, and ubiquinone-10 and 5-demethoxyubiquinone-10 contained in the ubiquinone-10 solution and 5-demethoxyubiquinone-10 solution after 30 cycles of operation are shown in Table 3. The concentrations of ubiquinone-10 and 5-demethoxyubiquinone-10 were measured by the same method as the HPLC analysis described in Example 1.

**[Table 2]**

| | | starting material solution feed valve | | | | eluent feed valve | | | | ubiquinone-10 withdrawal valve | | | | 5-demethoxyubiquinone-10 withdrawal valve | | | |
|---|---|---|---|---|---|---|---|---|---|---|---|---|---|---|---|---|---|
| process | step | F1 | F2 | F3 | F4 | W1 | W2 | W3 | W4 | A1 | A2 | A3 | A4 | L1 | L2 | L3 | L4 |
| 1 | feed withdrawal | ○ | × | × | × | × | × | × | × | ○ | × | × | × | × | × | × | × |
| | feed withdrawal | × | × | × | × | × | × | ○ | × | ○ | × | × | × | × | × | × | × |
| | feed withdrawal | × | × | × | × | × | × | ○ | × | × | × | × | × | × | × | ○ | × |
| | circulation | × | × | × | × | × | × | × | × | × | × | × | × | × | × | × | × |
| 2 | feed withdrawal | × | ○ | × | × | × | × | × | × | × | ○ | × | × | × | × | × | × |
| | feed withdrawal | × | × | × | × | × | × | × | ○ | × | ○ | × | × | × | × | × | × |
| | feed withdrawal | × | × | × | × | × | × | × | ○ | × | × | × | × | × | × | × | ○ |
| | circulation | × | × | × | × | × | × | × | × | × | × | × | × | × | × | × | × |
| 3 | feed withdrawal | × | × | ○ | × | × | × | × | × | × | × | ○ | × | × | × | × | × |
| | feed withdrawal | × | × | × | × | ○ | × | × | × | × | × | ○ | × | × | × | × | × |
| | feed withdrawal | × | × | × | × | ○ | × | × | × | × | × | × | × | ○ | × | × | × |
| | circulation | × | × | × | × | × | × | × | × | × | × | × | × | × | × | × | × |
| 4 | feed withdrawal | × | × | × | ○ | × | × | × | × | × | × | × | ○ | × | × | × | × |
| | feed withdrawal | × | × | × | × | × | ○ | × | × | × | × | × | ○ | × | × | × | × |
| | feed withdrawal | × | × | × | × | × | ○ | × | × | × | × | × | × | × | ○ | × | × |
| | circulation | × | × | × | × | × | × | × | × | × | × | × | × | × | × | × | × |

**[Table 3]**

| | ubiquinone-10 | | 5-demethoxyubiquinone-10 | |
|---|---|---|---|---|
| | concentration (g/L) | yield (%) | concentration (g/L) | yield (%) |
| starting material solution | 48.2 | 100.0 | 0.40 | 100.0 |
| ubiquinone-10 solution | 7.8 | 99.8 | 0.0 | 0.0 |
| 5-demethoxy-ubiquinone-10 solution | 0.0 | 0.2 | 0.04 | 100.0 |

As shown in Table 3, it has been clarified that ubiquinone-10 free of 5-demethoxyubiquinone-10, which is a substance structurally similar to ubiquinone-10, can be obtained in a yield of 99.8% by the method of the present invention. That is, the method of the present invention removes 5-demethoxyubiquinone, which is a substance structurally similar to ubiquinone-10, by 100%, and can purify ubiquinone-10 efficiently.

### Industrial Applicability

The present invention can be used for separating a substance structurally similar to ubiquinone-10 contained in a starting material solution containing ubiquinone-10 at high efficiency.

## Claims

1. A method of purifying ubiquinone-10, which comprises separating and removing a substance structurally similar to ubiquinone-10 contained in a starting material solution containing ubiquinone-10 by the use of a porous crosslinked acrylic polymer.

2. A method of purifying ubiquinone-10 by separating and removing a substance structurally similar to ubiquinone-10 contained in a starting material solution, which comprises feeding a starting material solution containing ubiquinone-10 in a simulated moving bed chromatography apparatus wherein a solution can circulate in one direction in a packed-bed in which a porous crosslinked acrylic polymer is packed, and moving an eluent in the packed-bed in one direction.

3. The method according to claim 1 or 2, wherein the porous crosslinked acrylic polymer is a spherical polymer particle having an average particle size of 10 - 500 µm.

4. The method according to any one of claims 1 to 3, wherein the porous crosslinked acrylic polymer is a polymer of methacrylic acid ester and ethyleneglycol dimethacrylate.

5. The method according to any one of claims 1 to 4, wherein the starting material solution containing ubiquinone-10 is an extract of a culture of a microorganism having an ability to produce ubiquinone-10, or a crude purified product of ubiquinone-10.

6. The method according to any one of claims 1 to 5, wherein the substance structurally similar to ubiquinone-10 is 5-demethoxyubiquinone-10.
